# EUROPÄISCHE PATENTANMELDUNG

(11) **EP 0 867 170 A2**
(43) Veröffentlichungstag der Anmeldung: **30.09.1998**
(21) Anmeldenummer: 98101496.2
(22) Anmeldetag: 29.01.1998
(51) Int. Cl.: A61K 7/13

(54) **Mittel und Verfahren zur Färbung keratinischer Fasern**

(30) Priorität: 26.03.1997 DE 19712649
(71) Anmelder: Wella Aktiengesellschaft, 64274 Darmstadt (DE)
(72) Erfinder: Braun, Hans-Jürgen, Dr., 3182 Überstorf (CH); Semadeni, Pascal André, Dr., 1792 Cordast (CH)

(57) **Zusammenfassung**

Die vorliegende Erfindung betrifft ein Mittel zum Färben von Fasern, insbesondere menschlicher Haare, welches dadurch gekennzeichnet ist, daß es eine Kombination aus mindestens einem zyklischen oxosubstituierten Endiol gemäß der allgemeinen Formel (I) und mindestens einer Verbindung aus der Gruppe bestehend aus Malonsäuredinitril, Maleinsäurediaminodinitril und Nitrobenzolderivaten enthält, wobei in Formel (I) R₁ und R₂ verschieden oder gleich sein können und unabhängig voneinander ein Wasserstoffatom oder ein Alkalimetallatom darstellen können, oder R₁ und R₂ gemeinsam ein Erdalkaliatom darstellen können und n gleich 0, 1, 2 oder 3 ist, sowie ein Verfahren zur Färbung von Haaren unter Verwendung dieses Mittels.

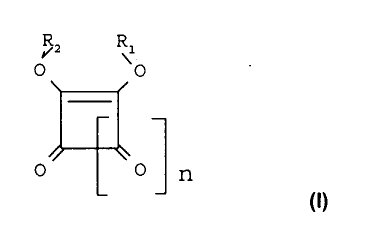

## Beschreibung

Die vorliegende Erfindung betrifft ein Mittel zur Färbung von natürlichen oder synthetischen Fasernmit einem Gehalt an zyklischen oxosubstituierten Endiolen und Malonsäuredinitril, Malonsäurediaminodinitril oder Nitrobenzolen sowie ein Verfahren zur Färbung von Fasern unter Verwendung dieses Mittels.

Färbesysteme auf der Basis von oxosubstituierten Endiolen sind aus der DE-OS 43 35 626 bekannt. Gemäß der DE-OS 43 35 626 sollen diese Verbindungen in Kombination mit Verbindungen mit einer primären oder sekundären Aminogruppe oder einer 1 Stickstoff enthaltenden heterozyklischen Verbindung oder einer aromatischen Hydroxyverbindung besonders brillante Farben im Gelb-, Braun-, Grün- und Violett-Bereich ergeben. Die beiden reaktiven Komponenten werden hierbei miteinander vermischt, bis auf Siedetemperatur erhitzt und die abgekühlte Färbelösung filtriert. Dieses Verfahren ist kompliziert und ergibt ohne Erhitzen wenig befriedigende orange Farbnuancen.

Es bestand daher die Aufgabe, Färbemittel sowie ein Verfahren zum Färben von Fasern zur Verfügung zu stellen, welche auf einfache Weise intensive Färbungen ermöglichen.

Es wurde nunmehr überraschend gefunden, daß diese Aufgabe durch die Verwendung einer Kombination von zyklischen oxosubstituierten Endiolen gemäß der allgemeinen Formel (I) mit Malonsäuredinitril, Maleinsäurediaminodinitril oder Nitrobenzolderivaten gelöst werden kann und die geschilderten Nachteile umgangen werden können.

Gegenstand der vorliegenden Erfindung ist daher ein Mittel zum Färben von natürlichen oder synthetischen Fasern, insbesondere von keratinischen Fasern, wie zum Beispiel Wolle, Pelzen oder menschlichen Haaren, mit einem Gehalt an einer Kombination aus mindestens einem zyklischen oxosubstituierten Endiol gemäß der allgemeinen Formel (I) und mindestens einer Verbindung aus der Gruppe bestehend aus Malonsäuredinitril, Maleinsäurediaminodinitril und Nitrobenzolderivaten, wobei in Formel (I) R₁ und R₂ verschieden oder gleich sein können und unabhängig voneinander ein Wasserstoffatom oder ein Alkalimetallatom darstellen können oder R₁ und R₂ gemeinsam ein Erdalkalimetallatom darstellen können und n gleich 0, 1, 2 oder 3 ist.

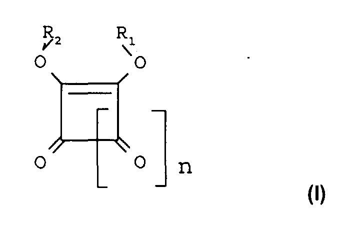

Bevorzugte zyklische oxosubstituierte Endiole gemäß der allgemeinen Formel (I) sind solche mit n gleich 2 oder 3, das heißt Dihydroxy-trioxo-cyclopenten (Krokonsäure), Dihydroxy-tetraoxo-cyclohexen (Rhodizonsäure) und deren Alkalimetallsalze oder Erdalkalimetallsalze.

Die zyklischen oxosubstituierten Endiole der Formel (I) sind in dem erfindungsgemäßen Färbemittel vorzugsweise in einer Gesamtmenge von 0,01 und 10 Gewichtsprozent, insbesondere in einer Gesamtmenge von 0,1 und 5 Gewichtsprozent enthalten.

Die zyklischen oxosubstituierten Endiole gemäß der allgemeinen Formel (I) ergeben in Kombination mit mindestens einer Verbindung aus der Gruppe bestehend aus Malonsäuredinitril, Maleinsäurediaminodinitril und Nitrobenzolderivaten auf natürlichen oder synthetischen Fasern intensive und modische Brauntöne, Rottöne und Schwarztöne, wobei die Gesamtmenge der Verbindungen aus der Gruppe bestehend aus Malonsäuredinitril, Maleinsäurediaminodinitril und Nitrobenzol-derivaten gleich 0,01 bis 10 Gewichtsprozent, insbesondere 0,1 bis 5 Gewichtsprozent ist.

Als geeignete Nitrobenzolderivate können hierbei 1,4-Bis[(2'-hydroxyethyl)amino]-2-nitrobenzol, 1-(2'-Hydroxyethyl)amino-2-nitro-4-bis-(2''-hydroxyethyl)amino-benzol (HC Blue No. 2), 1-Amino-3-methyl-4-(2'-hydroxyethyl)amino-6-nitro-benzol (HC Violet No. 1), 4,N-Ethyl,N-(2''-hydroxyethyl)amino-1-(2''-hydroxyethyl)amino-2-nitro-benzol-hydrochlorid (HC Blue No. 12), 4-Bis-(2'-hydroxyethyl)amino-1-(2''-methoxyethyl)amino-2-nitrobenzol HC Blue No. 11), 1-(2',3'-Dihydroxypropyl)amino-2-nitro-4-[N-methyl-(2''-hydroxyethyl)-amino]-benzol-Hydrochlorid (HC Blue No. 10), 1-[(2',3'-Dihydroxypropyl)amino]-2-nitro-4-[N-ethyl-2''-(hydroxyethyl)-amino]-benzol-Hydrochlorid (HC Blue No. 9), 1-(3'-Hydroxypropylamino)-2-nitro-4-bis-(2''-hydroxyethylamino)-benzol (HC Violet No. 2), 4,N-Methyl,N-(2',3'-dihydroxypropyl)amino-1-methylamino-2-nitro-benzol-Hydrochlorid (HC Blue No. 6), 4'Amino-2'-nitro-2''-carboxy-4''-dimethylamino-diphenylamin (HC Blue No. 13), 1-Amino-4-(2'-hydroxyethyl)-amino-2-nitrobenzol (HC Red No. 7), 4-Amino-2-nitro-diphenylamin (HC Red No. 1),1-Amino-2-nitro-4-bis-(2'-hydroxyethyl)-amino-benzol-Hydrochlorid (HC Red No. 13), 1-Amino-2-nitro-4-(2'-hydroxyethyl)amino-5-chlorbenzol, 1-(2'-Hydroxyethyl)amino-2-nitro-4-amino-benzol (HC Red No. 3), 1-Hydroxy-3-nitro-4-amino-benzol, 1-Hydroxy-3-nitro-4-(2'-hydroxyethylamino)benzol, 1-(2'-Aminoethyl)amino-2-nitro-4-(2'-hydroxyethoxy)-benzol (HC Orange No. 2), 3-Nitro-4-(2'-hydroxyethyl)amino-phenylglycerinether (HC Orange No. 3), 1-Amino-5-chlor-4-(2',3'-dihydroxypropyl)amino-2-nitro-benzol (HC Red No. 10), 1,4-Bis-[(2',3'-dihydroxypropyl)amino]-5-chlor-2-nitro-benzol (HC Red No. 11), 1-Hydroxy-2-(2'hydroxyethyl)amino-4,6-dinitro-benzol, 3-Nitro-4-ethylamino-benzoesäure, 4-Amino-2-nitro-diphenylamino-2-carbonsäure, 2-Chlor-6-ethylamino-4-nitrophenol, 2-Amino-6-chlor-4-nitrophenol, 1-Hydroxy-3-nitro-4-(3'-hydroxypropylamino)-benzol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red 14), 1-Amino-2-(2'-hydroxyethyl)amino-5-nitro-benzol (HC Yellow No. 5), 1-(2'-Hydroxyethoxy)-2-(2''-hydroxyethyl)amino-5-nitro-benzol (HC Yellow No. 4), 1-(2'-Hydroxyethyl)-amino-2-nitro-benzol (HC Yellow No. 2), 1-Methoxy-2-(2'-hydroxyethyl)-amino-5-nitro-benzol, 1-Hydroxy-2-amino-3-nitro-benzol, 1-Amino-2-methyl-6-nitro-benzol, 1-(2'Hydroxyethyl)-oxy-3-methylamino-4-nitro-benzol, 1-Methylamino-2-nitro-5-(2',3'-dihydroxypropyl)-oxybenzol, 1-(2'-Hydroxyethyl)amino-2-hydroxy-4-nitro-benzol (HC Yellow No. 11), 1-Methoxy-3-(2'-aminoethyl)-amino-4-nitro-benzol-Hydrochlorid (HC Yellow No.9), 1-(2'-Ureidoethyl)amino-4-nitro-benzol, 4-(2',3'-Dihydroxypropyl)-amino-3-nitro-trifluormethyl-benzol (HC Yellow No. 6), 2,4-Bis-[N-(2'-hydroxyethyl)amino]-5-chlor-nitrobenzol (HC Yellow No. 10), 4-(2'-Hydroxyethyl)amino-3-nitro-methylbenzol, 4-(2'-Hydroxyethyl)amino-3-nitro-chlorbenzol (HC Yellow No. 12), 4-(2'-Hydroxyethyl)amino-3-nitro-trifluormethyl-benzol (HC Yellow No. 13), 4-(2'-Hydroxyethyl)amino-3-nitro-benzonitril (HC Yellow No. 14), 4-(2'-Hydroxyethyl)amino-3-nitro-benzamid (HC Yellow No. 15) und insbesondere Nitroanilinderivate, beispielsweise 4-(Di(2-hydroxyethyl)amino)-2-nitroanilin, N-(2-Hydroxyethyl)-4-methyl-2-nitroanilin, 4-(2',3'-Dihydroxypropyl)-amino-3-nitro-trifluormethylbenzol, 4-(2'-Hydroxyethyl)-amino-3-nitro-chlorbenzol, 1-Hydroxy-2-amino-4,6-dinitro-benzol und 4-(2'-Hydroxyethyl)-amino-3-nitro-methylbenzol, genannt werden.

Zur Erzielung weiterer Farbnuancen können zusätzlichn übliche direktziehende Farbstoffe, beispielsweise Azofarbstoffe, Anthrachinonfarbstoffe oder Triphenylmethanfarbstoffe, alleine oder im Gemisch miteinander, zugesetzt werden. Beispiele für geeignete Azofarbstoffe sind 1-(4'-Nitrophenylazo)-2-methyl-4-bis-(2'-hydroxyethyl)amino-benzol, 1-(3'-Nitro-4-amino)-phenylazo-2-hydroxy-7-trimethyl-ammoniumchlorid-naphthalin, 1-(2'Hydroxy-4'sulfo-6'nitro)-naphthylazo-2-hydroxynaphthalin CI 15700, 1-(4'-Aminophenylazo)-2-methyl-4-bis-[(2'-hydroxyethyl)-amino]-benzol, 5-(4'-Dimethylaminophenylazo)-1,4-dimethyl-triazoniumchlorid, 1-(2'-Methoxyphenylazo)-2-hydroxy-7-trimethylammonium-naphthalinchorid, 1-(4'-Aminophenylazo)-2-hydroxy-7-trimethylammonium-naphthalin, 4-(3'-Trimethylammoniumphenylazo)-N-phenyl-3-methyl-pyrazolon (5), 4-Hydroxy-3-[(4'-sulfo-1'-naphthyl)azo]-1-naphthalinsulfonsäure, 1-(4'-Sulfophenylazo)-2-hydroxynaphthalin, 1-(4'-Sulfonphenylazo)-2-hydroxy-6-sulfo-naphthalin CI 15985, 4-Amino-[4'-bis-(2''-hydroxyethyl)amino]-azobenzol, 4-Amino-[4'-bis-(2''-hydroxyethyl)amino]-2'-methyl-azobenzol, 3-(2',6'-Diaminopyridyl-3'-azo)-pyridin, 7-Phenylazo-1-amino-3,6-disulfo-8-hydroxy-naphthalin, 5-Acetylamino-4-hydroxy-3-[(2'-methylphenyl)azo]-2,7-naphthalin-disulfonsäure und 2-(2',4'-Dimethylphenylazo)-6-(4''-sulfophenylazo)-1,3-dihydroxybenzol.

Beispiele für geeignete Anthrachinonfarbstoffe sind 1,4-Bis-(2',3'-dihydroxypropyl)amino-anthrachinon, 1-Methylamino-4-(2'-hydroxyethyl)amino-anthrachinon, 2-(2'-Aminoethyl)-amino-anthrachinon, 2-Brom-4,8-diamino-6-(3'-trimethylammonium)-phenylamino-1,5-naphthochinon, 1-(2'-Sulfo-4'-methyl-phenyl)-amino-4-hydroxy-anthrachinon, 1,4-Diamino-anthrachinon, 1-Amino-2-sulfo-4-cyclohexylamino-anthrachinon, 1-Methylamino-4-aminopropylaminoanthrachinon, 1-Aminopropylamino-anthrachinon, 1,4-Diamino-2-methoxy-anthrachinon und 1,4-Bis(2-Hydroxyethyl)amino-5,8-dihydroxy-anthrachinon.
Beispiele für geeignete Triphenylmethanfarbstoffe sind 4',4'',4'''-Triamino-3-methyl-triphenylcarboniumchlorid, Bis-(4,4-Diethylaminophenyl)-4'-ethylamino-naphthyl-carboniumchlorid, Bis-(4,4-Dimethylaminophen)-4'-phenylamino-naphthyl-carbonsiumchlorid (Basic Blue 26, CI 44045) und 4,4-Bis-(N-Ethyl-3-sulfobenzyl)-amino-2''-sulfofuchsonium.

Die Gesamtmenge der zugesetzten direktziehenden Farbstoffe beträgt etwa 0,01 bis 5 Gewichtsprozent, vorzugsweise etwa 0,1 bis 4 Gewichtsprozent.

Das erfindungsgemäße Färbemittel kann grundsätzlich sowohl zum Färben von Naturfasern, wie zum Beispiel Keratinfasern (Wolle, Pelze, menschliche Haare), Baumwolle, Jute, Sisal, Leinen oder Seide, modifizierten Naturfasern, wie zum Beispiel Regenerat-cellulose, Nitrocellulose, Alkylcellulose oder Hydroxyalkylcellulose, als auch synthetischen Fasern, wie zum Beispiel Polyamidfasern oder Polyurethanfasern verwendet werden. Vorzugsweise wird das erfindungsgemäße Färbemittel zur Färbung von keratinischen Fasern, insbesondere menschlichen Haaren verwendet.

Das Färbemittel kann in Form einer wäßrigen oder wäßrig-alkoholischen Lösung, einer Creme, eines Gels, einer tensidhaltigen schäumenden Lösung (Shampoo, Aerosol) oder einen Emulsion vorliegen, wobei seine Zusammensetzung eine Mischung der Farbstoffkomponenten mit den für solche Zubereitungen üblichen kosmetischen Zusätzen darstellt.

Übliche kosmetische Zusätze sind zum Beispiel Lösungsmittel wie Wasser, niedere aliphatische einwertige oder mehrwertige Alkohole, deren Ester und Ether, beispielsweise Alkanole, insbesondere mit 1 bis 4 Kohlenstoffatomen im Molekül, zum Beispiel Ethanol, Propanol, Isopropanol, Butanol oder Isobutanol, oder zweiwertige und dreiwertige Alkohole, insbesondere solche mit 2 bis 6 Kohlenstoffatomen im Molekül, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol oder Dipropylenglycol, Polyalkylenglycole wie Triethylenglycol, Polyethylenglycol, Tripropylenglycol und Polypropylenglycol, niedere Alkylether von mehrwertigen Alkoholen, wie zum Beispiel Ethylenglycolmonomethylether, Ethylenglycolmonoethylether, Ethylenglycolmonopropylether, Ethylenglycolmonobutylether, Diethylenglycolmonomethylether, Diethylenglycolmonoethylether, Triethylenglycolmonomethylether oder Triethylenglycolmonoethylether, Ketone und Ketoalkohole, insbesondere solche mit 3 bis 7 Kohlenstoffatomen im Molekül, wie zum Beispiel Aceton, Methylethylketon, Diethylketon, Methylisobutylketon, Methylphenylketon, Cyclopentanon, Cyclohexanon oder Diacetonalkohol, Ether, wie zum Beispiel Dibutylether, Tetrahydrofuran, Dioxan oder Diisopropylether, Ester wie zum Beispiel Ethylformiat, Methylformiat, Methylacetat, Ethylacetat, Propylenacetat, Butylacetat, Phenylacetat, Ethlglycolmonoethyletheracetat oder Essigsäurehydroxyethylester, Amide wie zum Beispiel Dimethylformamid, Dimethylacetamid oder N-Methylpyrrolidon, ferner Harnstoff, Tetramethylharnstoff und Thiodiglycol. Weiterhin können dem erfindungsgemäßen Färbemittel Netzmittel oder Emulgatoren aus den Klassen der anionischen, kationischen, amphoteren, nichtionogenen oder zwitterionischen oberflächenaktiven Substanzen, beispielsweise Fettalkoholsulfate, Alkansulfonate, Alkylbenzolsulfonate, Alkyltrimethylammoniumsalze, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate oder Alkylpolyglucoside; Verdickungsmittel wie höhere Fettalkohole, Stärke, Cellulosederivate, Vaseline, Paraffnöle oder Fettsäuren und andere Fettkomponenten in emulgierter Form, wasserlösliche polymere Verdickungsmittel wie natürliche Gummen, Guargummi, Xanthangummi, Johannisbrotkernmehl, Pektin, Dextran, Agar-Agar, Amylose, Amylopektin, Dextrine oder Tone und vollsynthetische Hydrokolloide wie Polyvinylalkohol, außerdem Pflegestoffe wie Lanolinderivate, Cholesterin, Pantothensäure, wasserlösliche kationische Polymere, Proteinderivate, Provitamine, Vitamine, Pflanzenextrakte, Zucker oder Betain, Hilfsstoffe wie Elektrolyte, Antioxidantien, Fettamide, Sequestrierungsmittel, filmbildende Agentien und Konservierungsmittel zugesetzt werden.

Die genannten kosmetischen Zusatzstoffe werden in den für solche Zwecke üblichen Mengen verwendet, zum Beispiel die Netzmittel und Emulgatoren in einer Gesamtmenge von etwa 0,5 bis 30 Gewichtsprozent, die Verdicker in einer Gesamtmenge von etwa 0,1 bis 25 Gewichtsprozent und die Pflegestoffe in einer Gesamtmenge von etwa 0,1 bis 5 Gewichtsprozent.

Das erfindungsgemäße Mittel zur Färbung keratinischer Fasern besitzt einen pH-Wert von etwa 0,3 und 6,5 auf, wobei, falls erforderlich, die Einstellung dieses pH-Wertes vorzugsweise mit schwachen organischen Säuren, wie zum Beispiel Zitronensäure, Glykolsäure, Milchsäure, Äpfelsäure, Ascorbinsäure oder Weinsäure erfolgt.

Das erfindungsgemäße Mittel liegt vorzugsweise in Form einer 2-Komponenten-Zubereitung vor, aus der unmittelbar vor der Anwendung durch Vermischen einer wäßrigen oder wässerig-alkoholischen Lösung, welche mindestens eine Verbindung aus der Gruppe bestehend aus Malonsäuredinitril, Maleinsäurediaminodinitril und Nitrobenzolen enthält, (Komponente 1) mit mindestens einem zyklischen oxosubstituierten Endiol gemäß der allgemeinen Formel (I) (Komponente 2) das gebrauchsfertige Färbemittel hergestellt wird.

Zur Haarfärbung trägt man eine für die Behandlung ausreichende Menge, je nach Haarfülle im allgemeinen etwa 10 bis 60 g, des erfindungsgemäßen Färbemittels auf das Haar auf. Man läßt das Gemisch bei 15 bis 50 °C etwa 10 bis 60 Minuten lang, vorzugsweise etwa 20 bis 45 Minuten bei 25 bis 40 °C, auf das Haar einwirken, spült sodann das Haar mit Wasser aus und trocknet es. Gegebenenfalls wird im Anschluß hieran mit einem Shampoo gewaschen und/oder mit einer schwachen organischen Säure, wie zum Beispiel Zitronensäure, Glykolsäure, Milchsäure, Äpfelsäure, Ascorbinsäure oder Weinsäure nachgespült. Anschließend wird das Haar getrocknet.

Das Färbemittel besitzt bereits bei Temperaturen unterhalb von 40 °C ein sehr gutes Penetrationsvermögen auf menschliche Haare und ergibt eine sehr gute Deckkraft vom Haaransatz bis zu den Haarspitzen. Hinsichtlich der färberischen Möglichkeiten ermöglicht das erfindungsgemäße Färbemittel je nach Art und Zusammensetzung der Farbkomponenten eine breite Palette verschiedener modischen Farbnuancen im Rotbereich bis hin zum Braunbereich und Schwarzbereich. Bemerkenswert ist hierbei die große Farbintensität und die Farbreinheit der erzielten Färbungen

Die nachfolgenden Beispiele sollen den Erfindungsgegenstand näher erläutern ohne ihn hierauf zu beschränken

### Beispiele

### Beispiel 1: Haarfärbemittel mit Kaliumrhodizonat

- 0,165 g: Malonsäuredinitril
- 1,000 g: Isopropanol
- 0,100 g: Benzylalkohol
- 0,500 g: Milchsäure
- 7,620g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert des gebrauchsfertigen Haarfärbemittels beträgt 2,9. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine cognac-braune Färbung.

### Beispiel 2: Haarfärbemittel mit Kaliumrhodizonat

- 0,270 g: Maleinsäurediaminodinitril
- 1,000 g: Isopropanol
- 0,100 g: Benzylalkohol
- 0,500 g: Milchsäure
- 7,515 g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 3,3. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine rehbraune Färbung.

### Beispiel 3: Haarfärbemittel mit Kaliumrhodizonat

- 0,603 g: 4-(Di(2-hydroxyethyl)amino)-2-nitroanilin
- 1,000 g: Isopropanol
- 0,100 g: Benzylalkohol
- 0,500 g: Milchsäure
- 7,182g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 3,0. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine dunkelviolette-braunschwarze Färbung.

### Beispiel 4: Haarfärbemittel mit Krokonsäure

- 0,165 g: Malonsäuredinitril
- 1,000 g: Isopropanol
- 0,100 g: Benzylalkohol
- 0,500 g: Milchsäure
- 7,880 g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 0,3. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine dunkelbraun-bordeauxrote Färbung.

### Beispiel 5: Haarfärbemittel mit Krokonsäure

- 0,270 g: Maleinsäurediaminodinitril
- 1,000 g: Isopropanol
- 0,100 g: Benzylalkohol
- 0,500 g: Milchsäure
- 7,775g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 0,4. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine dunkle nussbraune Färbung.

### Beispiel 6: Haarfärbemittel mit Krokonsäure

- 0,603 g: 4-(Di(2-hydroxyethyl)amino)-2-nitroanilin
- 1,000 g: Isopropanol
- 0,100 g: Benzylalkohol
- 0,500 g: Milchsäure
- 7,442 g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 6,0. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine rötlich-dunkelbraune Färbung.

### Beispiel 7: Haarfärbemittel mit Kaliumrhodizonat

- 0,245 g: N-(2-Hydroxyethyl)-4-methyl-2-nitroanilin
- 1,000 g: Isopropanol
- 0,100 g: Benzylalkohol
- 0,500 g: Milchsäure
- 7,540 g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 6,0. Gebleichtes Haar wird sodann 40 Minuten bei einer Temperatur von 40° C mit diesem Haarfärbemittel behandelt. Anschließend wird das Haar mit Wasser gespült und getrocknet. Anschließend wird das Haar mit Wasser gespült und getrocknet. Es resultiert eine intensive, orange Färbung.

### Beispiel 8: Haarfärbemittel mit Krokonsäure

- 0,245 mg: N-(2-Hydroxyethyl)-4-methyl-2-nitroanilin
- 1,000 g: Isopropanol
- 0,100 g: Benzylalkohol
- 0,500 g: Milchsäure
- 7,800 g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 6,0. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine orange Färbung.

### Beispiel 9: Haarfärbemittel mit Kaliumrhodizonat

- 0,892 g: 1-((4-Aminophenyl)azo)-7-(trimethylammonium)-2-naphthol
- 0,270 g: Maleinsäurediaminodinitril
- 1,000 g: Isopropanol
- 0,100 g: Benzylalkohol
- 0,500 g: Milchsäure
- 6,623 g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 2,7. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine natürliche kastanienbraune Färbung.

### Beispiel 10: Haarfärbemittel mit Krokonsäure

- 0,892 g: 1-((4-Aminophenyl)azo)-7-(trimethylammonium)-2-naphthol
- 0,270 g: Maleinsäurediaminodinitril
- 1,000g: Isopropanol
- 0,100 g: Benzylalkohol
- 0,500 g: Milchsäure
- 6,883 g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 2,9. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine natürliche kastanienbraune Färbung.

### Beispiel 11: Haarfärbemittel mit Kaliumrhodizonat

- 0,596 g: 1,4-Diamino-9,10-anthracendion
- 0,165 g: Malonsäuredinitril
- 1,000 g: Isopropanol
- 0,100 g: Benzylalkohol
- 0,500 g: Milchsäure
- 7,024 g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 3,0. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine rehbraune Färbung.

### Beispiel 12: Haarfärbemittel mit Krokonsäure

- 0,596 g: 1,4-Diamino-9,10-Anthracendion
- 0,165 g: Malonsäuredinitril
- 1,000 g: Isopropanol
- 0,100 g: Benzylalkohol
- 0,500 g: Milchsäure
- 7,284 g: Wasser

Die vorstehende Farbträgermasse wird unmittelbar vor Gebrauch mit 0,355 g Krokonsäure zum gebrauchsfertigen Haarfärbemittel vermischt.

Der pH-Wert dieses Mittels ist gleich 0,8. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine modische, kräftige rötlichbraune Färbung.

### Beispiel 13: Haarfärbemittel mit Kaliumrhodizonat

- 1,284 g: Di(4-(diethylamino)phenyl)(4-(ethylamino)-naphthyl)carbeniumchlorid
- 0,270 g: Maleinsäurediaminodinitril
- 1,000 g: Isopropanol
- 0,100 g: Benzylalkohol
- 0,500 g: Milchsäure
- 6,231 g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 2,7. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine blaugraue dunkle Färbung.

### Beispiel 14: Haarfärbemittel mit Krokonsäure

- 1,284 g: Di(4-(diethylamino)phenyl)(4-(ethylamino)-naphthyl)carbeniumchlorid
- 0,270 g: Maleinsäurediaminodinitril
- 1,000 g: Isopropanol
- 0,100 g: Benzylalkohol
- 0,500 g: Milchsäure
- 6,491 g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 3,0. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine olivgrüne Färbung.

### Beispiel 15: Haarfärbemittel mit Kaliumrhodizonat

- 0,249 g: 1-Hydroxy-2-amino-4,6-dinitro-benzol
- 0,270 g: Maleinsäurediaminodinitril
- 1,000 g: Isopropanol
- 0,100 g: Benzylalkohol
- 0,500 g: Milchsäure
- 7,266 g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 3,4. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine natürliche rötlichschimmernde schwarze Färbung.

### Beispiel 16: Haarfärbemittel mit Krokonsäure

- 0,249 g: 1-Hydroxy-2-amino-4,6-dinitro-benzol
- 0,165 g: Malonsäuredinitril
- 1,000 g: Isopropanol
- 0,100 g: Benzylalkohol
- 0,500 g: Milchsäure
- 7,631 g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 0,7. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine tiefschwarze Färbung.

### Beispiel 17: Haarfärbemittel mit Kaliumrhodizonat

- 0,197 g: 4-((2-hydroxyethyl)amino)-2-nitroanilin
- 0,270 g: Maleinsäurediaminodinitril
- 1,000 g: Isopropanol
- 0,100 g: Benzylalkohol
- 0,500 g: Milchsäure
- 7,318 g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 3,5. Gebleichtes Haar wird sodann 40 Minuten bei einer Temperatur von 40° C mit diesem Haarfärbemittel behandelt. Anschließend wird das Haar mit Wasser gespült und getrocknet.. Es resultiert eine natürliche rötlichbraunschwarze Färbung.

### Beispiel 18: Haarfärbemittel mit Krokonsäure

- 0,197 g: 4-((2-hydroxyethyl)amino)-2-nitroanilin
- 0,165 g: Malonsäuredinitril
- 1,000 g: Isopropanol
- 0,100 g: Benzylalkohol
- 0,500 g: Milchsäure
- 7,683 g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 1,5. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine rot-schwarze Färbung.

### Beispiel 19: Haarfärbemittel mit Kaliumrhodizonat

- 0,357 g: 4-(di(2-hydroxyethyl)amino)-N-(2-hydroxyethyl)-2-nitroanilin
- 0,270 g: Maleinsäurediaminodinitril
- 1,000 g: Isopropanol
- 0,100 g: Benzylalkohol
- 0,500 g: Milchsäure
- 7,158 g: Wasser

Die vorstehende Farbträgermasse wird unmittelbar vor Gebrauch mit 0,615 g Kaliumrhodizonat zum gebrauchsfertigen Haarfärbemittel vermischt. Der pH-Wert dieses Mittels ist gleich 3,2. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine natürliche rötlichschimmernde tiefschwarze Färbung.

### Beispiel 20: Haarfärbemittel mit Krokonsäure

- 0,357 g: 4-(di(2-hydroxyethyl)amino)-N-(2-hydroxyethyl)-2-nitroanilin
- 0,270 g: Maleinsäurediaminodinitril
- 1,000 g: Isopropanol
- 0,100 g: Benzylalkohol
- 0,500 g: Milchsäure
- 7,418 g: Wasser

Die vorstehende Farbträgermasse wird unmittelbar vor Gebrauch mit 0,355 g Krokonsäure zum gebrauchsfertigen Haarfärbemittel vermischt. Der pH-Wert dieses Mittels ist gleich 1,0. Gebleichtes Haar wird sodann 40 Minuten bei einer Temperatur von 40° C mit diesem Haarfärbemittel behandelt. Anschließend wird das Haar mit Wasser gespült und getrocknet. Es resultiert eine tiefschwarze Färbung.

Alle Prozentangaben stellen, soweit nicht anders angegeben, Gewichtsprozente dar.

## Patentansprüche

1. Mittel zum Färben von natürliche oder synthetischen Fasern, insbesondere menschlicher Haare, dadurch gekennzeichnet, daß es eine Kombination aus mindestens einem zyklischen oxosubstituierten Endiol gemäß der allgemeinen Formel (I) und mindestens einer Verbindung aus der Gruppe bestehend aus Malonsäuredinitril, Maleinsäurediaminodinitril und Nitrobenzolderivaten enthält, wobei in Formel (I) R₁ und R₂ verschieden oder gleich sein können und unabhängig voneinander ein Wasserstoffatom oder ein Alkalimetallatom darstellen können oder R₁ und R₂ gemeinsam ein Erdalkalimetallatom darstellen können und n gleich 0, 1, 2 oder 3 ist.

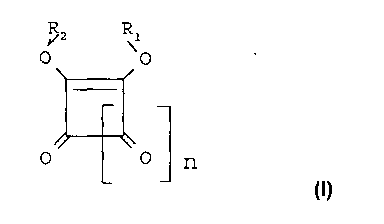

2. Mittel nach Anspruch 1, dadurch gekennzeichnet, daß die Verbindung der allgemeinen Formel (I) ausgewählt ist aus Dihydroxytrioxo-cyclopenten (Krokonsäure), Dihydroxy-tetraoxo-cyclohexen (Rhodizonsäure) und deren Alkalimetallsalzen oder Erdalkalimetallsalzen.

3. Mittel nach Anspruch 1 oder 2, dadurch gekennzeichnet, daß die Verbindung der allgemeinen Formel (I) in einer Gesamtmenge von 0,01 bis 10 Gewichtsprozent enthalten ist.

4. Mittel nach einem der Ansprüche 1 bis 3, dadurch gekennzeichnet, daß das Nitrobenzolderivat ausgewählt ist aus 1,4-Bis[(2'-hydroxyethyl)amino]-2-nitrobenzol, 1-(2'-Hydroxyethyl)amino-2-nitro-4-bis-(2''-hydroxyethyl)amino-benzol, 1-Amino-3-methyl-4-(2'-hydroxyethyl)amino-6-nitro-benzol, 4,N-Ethyl,N-(2''-hydroxyethyl)amino-1-(2''-hydroxyethyl)amino-2-nitro-benzol-hydrochlorid, 4-Bis-(2'-hydroxyethyl)amino-1-(2''-methoxyethyl)amino-2-nitrobenzol, 1-(2',3'-Dihydroxypropyl)amino-2-nitro-4-[N-methyl-(2''-hydroxyethyl)-amino]-benzol-Hydrochlorid, 1-[(2',3'-Dihydroxypropyl)amino]-2-nitro-4-[N-ethyl-2''-(hydroxyethyl)-amino]-benzol-Hydrochlorid, 1-(3'-Hydroxypropylamino)-2-nitro-4-bis-(2''-hydroxyethylamino)-benzol, 4,N-Methyl,N-(2',3'-dihydroxypropyl)amino-1-methylamino-2-nitro-benzol-Hydrochlorid, 4'Amino-2'-nitro-2''-carboxy-4''-dimethylamino-diphenylamin, 1-Amino-4-(2'-hydroxyethyl)-amino-2-nitrobenzol, 4-Amino-2-nitro-diphenylamin,1-Amino-2-nitro-4-bis-(2'-hydroxyethyl)amino-benzol-Hydrochlorid, 1-Amino-2-nitro-4-(2'-hydroxyethyl)amino-5-chlorbenzol, 1-(2'-Hydroxyethyl)amino-2-nitro-4-amino-benzol, 1-Hydroxy-3-nitro-4-amino-benzol, 1-Hydroxy-3-nitro-4-(2'-hydroxyethylamino)benzol, 1-(2'-Aminoethyl)amino-2-nitro-4-(2'-hydroxyethoxy)-benzol, 3-Nitro-4-(2'-hydroxyethyl)amino-phenylglycerinether, 1-Amino-5-chlor-4-(2',3'-dihydroxypropyl)amino-2-nitro-benzol, 1,4-Bis-[(2',3'-dihydroxypropyl)amino]-5-chlor-2-nitro-benzol, 1-Hydroxy-2-(2'hydroxyethyl)amino-4,6-dinitro-benzol, 3-Nitro-4-ethylamino-benzoesäure, 4-Amino-2-nitro-diphenylamino-2-carbonsäure, 2-Chlor-6-ethylamino-4-nitrophenol, 2-Amino-6-chlor-4-nitrophenol, 1-Hydroxy-3-nitro-4-(3'-hydroxypropylamino)-benzol, 2,5-Diamino-6-nitropyridin, 1,2,3,4-Tetrahydro-6-nitrochinoxalin, 7-Amino-3,4-dihydro-6-nitro-2H-1,4-benzoxazin, 1-Amino-2-(2'-hydroxyethyl)amino-5-nitro-benzol, 1-(2'-Hydroxyethoxy)-2-(2''-hydroxyethyl)amino-5-nitro-benzol, 1-(2'-Hydroxyethyl)amino-2-nitro-benzol, 1-Methoxy-2-(2'-hydroxyethyl)amino-5-nitro-benzol, 1-Hydroxy-2-amino-3-nitro-benzol, 1-Amino-2-methyl-6-nitro-benzol, 1-(2'Hydroxyethyl)-oxy-3-methylamino-4-nitro-benzol, 1-Methylamino-2-nitro-5-(2',3'-dihydroxypropyl)-oxybenzol, 1-(2'-Hydroxyethyl)amino-2-hydroxy-4-nitro-benzol, 1-Methoxy-3-(2'-aminoethyl)-amino-4-nitro-benzol-Hydrochlorid, 1-(2'-Ureidoethyl)amino-4-nitro-benzol, 4-(2',3'-Dihydroxypropyl)amino-3-nitro-trifluormethyl-benzol, 2,4-Bis-[N-(2'-hydroxyethyl)amino]-5-chlor-nitrobenzol, 4-(2'-Hydroxyethyl)amino-3-nitro-methylbenzol, 4-(2'-Hydroxyethyl)amino-3-nitro-chlorbenzol, 4-(2'-Hydroxyethyl)amino-3-nitro-trifluormethyl-benzol, 4-(2'-Hydroxyethyl)amino-3-nitro-benzonitril, 4-(2'-Hydroxyethyl)amino-3-nitro-benzamid, 4-(Di(2-hydroxyethyl)amino)-2-nitroanilin, N-(2-Hydroxyethyl)-4-methyl-2-nitroanilin, 4-(2',3'-Dihydroxypropyl)-amino-3-nitro-trifluormethylbenzol, 4-(2'-Hydroxyethyl)-amino-3-nitro-chlorbenzol, 1-Hydroxy-2-amino-4,6-dinitro-benzol und 4-(2'-Hydroxyethyl)-amino-3-nitro-methylbenzol.

5. Mittel nach einem der Ansprüche 1 bis 4, dadurch gekennzeichnet, daß das Malonsäuredinitril, Maleinsäurediaminodinitril und Nitrobenzolderivat in einer Gesamtmenge von 0,01 bis 10 Gewichtsprozent enthalten ist.

6. Mittel nach einem der Ansprüche 1 bis 5, dadurch gekennzeichnet, daß es für Haarfärbemittel übliche kosmetisch verträgliche Zusatzstoffe enthält.

7. Mittel nach einem der Ansprüche 1 bis 6, dadurch gekennzeichnet, daß es zusätzlich übliche direktziehende Farbstoffe aus der Gruppe der Azofarbstoffe, Anthrachinonfarbstoffe und Triphenylmethanfarbstoffe enthält.

8. Mittel nach einem der Ansprüche 1 bis 7, dadurch gekennzeichnet, daß es in Form einer 2-Komponenten-Zubereitung konfektioniert ist und unmittelbar vor der Anwendung durch Vermischen einer wäßrigen oder wässerig-alkoholischen Lösung, welche mindestens eine Verbindung aus der Gruppe bestehend aus Malonsäuredinitril, Maleinsäurediaminodinitril und Nitrobenzolen enthält, mit mindestens zyklischen oxosubstituierten Endiol gemäß der allgemeinen Formel (I) hergestellt wird.

9. Mittel nach einem der Ansprüche 1 bis 8, dadaurch gekennzeichnet, daß es einen pH-Wert von 0,3 bis 6,5 aufweist.

10. Verfahren zum Färben von Haaren, dadurch gekennzeichnet, daß man eine für die Färbung ausreichende Menge eines Mittels nach einem der Ansprüche 1 bis 9 auf das Haar aufträgt und nach einer Einwirkungszeit von 10 bis 60 Minuten bei 15° bis 50° C mit Wasser ausspült, gegebenenfalls mit einem Shampoo wäscht, und trocknet.
